# EUROPEAN PATENT SPECIFICATION

(11) **EP 0 707 858 B1**
(45) Date of publication and mention of the grant of the patent: **24.11.1999**
(21) Application number: 94203063.6
(22) Date of filing: 21.10.1994
(51) Int. Cl.: A61L 11/00, A61L 2/12

(54) **A process for disposing of refuse which includes pathogenic components**
Verfahren zur Beseitigung von pathogene Bestandteile enthaltendem Müll
Procédé pour l'évacuation d'ordures contenant des composés pathogènes

(43) Date of publication of application: 24.04.1996
(73) Proprietor: VOMM IMPIANTI E PROCESSI S.r.L., 20089 Rozzano (Milano) (IT)
(72) Inventor: Vezzani, Corrado, I-20146 Milano (IT)
(74) Representative: Vannini, Torquato

(56) References cited:
- WO-A-92/12738

## Description

In its most general aspect the present invention relates to a method for disposing of refuse which includes pathogenic and/or toxic components.

More particularly this invention relates to a method for the sterilisation and disposal of hospital refuse.

It is well known that the disposal of hospital refuse currently constitutes a serious problem from an environmental point of view. In fact, because of its pathogenic germ content such refuse cannot be disposed of via the usual discharge means authorised for the so-called urban refuse.

On the other hand the extremely varied nature of the composition of such refuse makes its disposal by incinerator means equally problematic.

As well as artificial, synthetic or vegetable fibre material (such as gauzes or cloths), for example sheets of the so-called one-time use type, such refuse contains rubber, metal and plastics materials as well as disinfectant residues and residues of other medical substances widely utilised in hospitals.

These components (in particular the plastics and disinfectants) often contain fluorinated compounds which have a well known tendency to generate compounds of the tetrachlorodibenzodioxin type following treatments at high temperature, such as are utilised in incinerators.

The risk and the noxious nature of these latter compounds, commonly called dioxins, is well known and constitutes the main reason why the use of incinerators for the disposal of hospital refuse is not acceptable.

For the reasons explained above the necessity of finding a method of disposal of hospital refuse which overcomes the problems of the environmental impact caused by the use of incinerators is currently very strongly felt.

In order, on the one hand, to overcome the disadvantages related to the use of incineration installations and, on the other hand, to overcome the risk of allowing the survival of pathogenic germs which are always present in hospital refuse, methods have been proposed which provide for the sterilisation of the hospital refuse before being sent to the normal urban refuse discharge.

In one of these methods the sterilisation is effected by subjecting successive charges of refuse to respective heat treatments in rotating drums in a discontinuous manner.

This method has, however, the disadvantage of a high fire risk because of the presence of cellulosic fibres and solvents in the materials to be treated.

This fire risk is not even eliminated by working in the absence of oxygen in that the peroxide groups present in the rubbers as a consequence of the vulcanisation process can act to support combustion in place of atmospheric oxygen.

Moreover, in this batch process the individual charges of refuse have a significant mass such that the heat treatment is necessarily non-uniform and consequently the complete sterilisation throughout the whole mass of the material is not guaranteed.

To overcome the recognised disadvantages of the batch process (the treatment of successive loads of large mass) attempts have been made to sterilise hospital refuse by utilising a technology based on the use of microwaves; even in this case there are serious problems related to the safety of the installation.

Micrometric waves at high energy (microwaves) utilised in such installations can in fact be extremely dangerous, for example as a result of uncontrollable and unpredictable reflection phenomena due to the presence of, for example, metal objects in the refuse to be treated. Because they require very strict safety measures and highly specialised personnel such installations involve very high management costs and notwithstanding this do not always give satisfactory results.

Patent application no. W092/12738 discloses a process for the continuous disposal of medical waste, including the steps of
a) submitting the waste to the action of steam and of disintegration means inside a sealed pressure vessel and
b) transferring the thus treated waste into a screw conveyor, wherein the waste is agitated and subjected to the sterilising action of steam while being moved towards the discharge end of the conveyor.

The problem on which the present invention is based is that of making available a method for the disposal of hospital refuse which can satisfy current requirements as explained more fully above, whilst contemporaneously overcoming the disadvantages discussed with reference to the prior art.

The technical solution of this problem is to subject small quantities of hospital refuse maintained in a highly turbulent state and constituting a substantially continuous flow of material to a sterilising heat treatment.

According to this idea the said technical problem is resolved according to the present invention by a method for disposal of hospital refuse and the like characterised by the fact that is comprises the steps of:
- grinding hospital refuse to obtain a comminuted material which can be pumped;
- sterilisation heat treatment of the said comminuted material disposed in a thin layer in contact with a heated wall and maintained in a highly turbulent condition
- cooling of the said sterilised comminuted material and disposal thereof by discharge.

Advantageously the method of this invention is put into practice by utilising sterilisation apparatus comprising a cylindrical tubular body provided with a heating jacket, an inlet opening for the material to be treated and a discharge opening for treated material, and a paddle rotor rotatably supported in the cylindrical body and driven to rotate at 200 - 1500 revolutions per minute.

Such apparatus will be identified hereafter in the description and in the subsequent claims with the term "turbosteriliser".

By utilising such apparatus the method of disposing of hospital refuse according to the present invention comprises the steps of:
- comminuting the said hospital refuse to obtain a pumpable comminuted material,
- supply of a continuous flow of this material to the inlet of a turbosteriliser having cylindrical internal walls heated to a temperature of 300 - 500°C and in which the paddle rotor is rotated at a speed lying between 400 and 1500 revolutions per minute,
- centrifuging of the said comminuted material to form a thin tubular layer which flows in contact with the said heated wall towards the said discharge aperture with contemporaneous sterilisation of the comminuted material,
- cooling and recovery of the sterilised and comminuted material and subsequently delivering it to a discharge outlet.

Contemporaneously with the heat sterilisation of the comminuted material maintained in a dynamic thin layer it is dried by the removal in the form of steam of the moisture originally contained in it, as well as the removal of all the substances which evaporate at the sterilisation temperature reached.

In accordance with a further characteristic of this invention the steam generated during the sterilisation heat treatment of the comminuted material is recovered, subjected to a further sterilisation at high pressure and condensed to be subsequently reincorporated into the sterilised comminuted material, with which it is delivered to the discharge.

Advantageously the condensed steam and sterilised comminuted material are intimately mixed and simultaneously cooled to obtain a paste which is subsequently subjected to compaction and briquetting and then discharged.

The advantages and the characteristics of this invention will become more apparent from the following description of an embodiment of a process for continuous sterilisation of hospital refuse, made from hereon with reference to the attached drawings by way of indication only.
Figure 1 is a schematic representation of apparatus for performing the process of the invention;
Figure 2 is a schematic representation of the apparatus used to perform a variant in the said process; and
Figure 3 schematically illustrates a complete installation for disposal of refuse containing pathogens, including the apparatus necessary for performing the process of the invention.

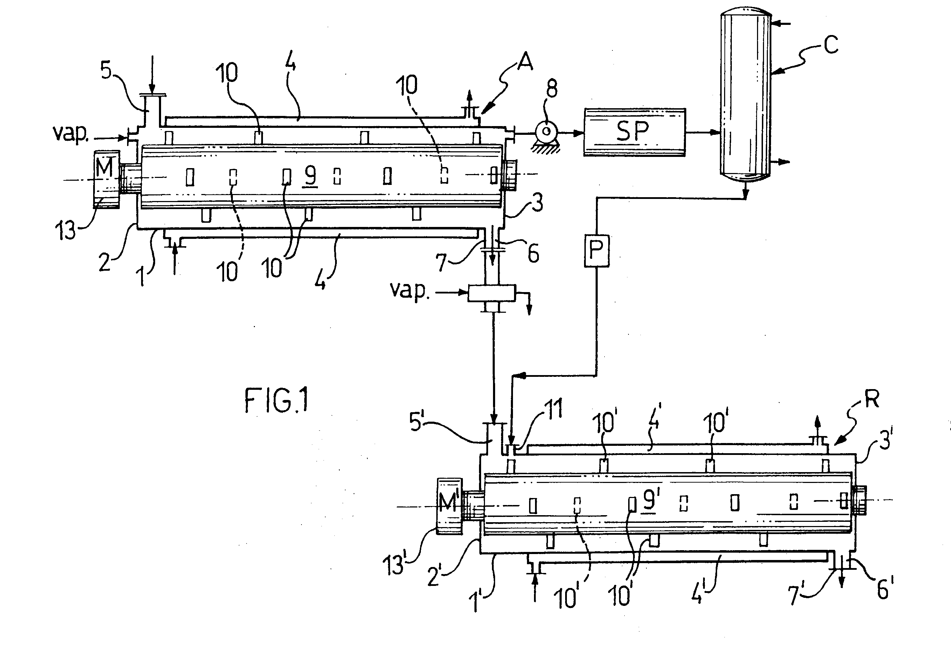

With reference to Figure 1 the apparatus utilised for the process according to the invention for continuous sterilisation of hospital refuse comprises first apparatus A which hereinafter in the description will be called a turbosteriliser, second apparatus R hereinafter called a turbocooler, a high pressure steriliser SP and a condenser C.

The turbosteriliser A essentially comprises a cylindrical tubular body 1 preferably disposed with its axis horizontal, closed at its opposite ends 2,3 and provided coaxially with a heating jacket 4 intended to receive appropriate heating means, for example an electrical resistance, a diathermic fluid or oil, or the like.

The tubular body 1 is provided with an inlet aperture 5 for comminuted material obtained by finely comminuting hospital refuse, and a discharge aperture 6 for the treated comminuted material.

This aperture 6 is in communication via a duct 7 with the inlet aperture 5' of the turbocooler R .

Rotatably supported in the tubular body 1 is a paddle rotor 9. The paddle 10 of this rotor are disposed helically and are orientated to centrifuge the comminuted material against the internal wall of the tubular body itself and simultaneously to convey this material towards the discharge aperture 6. A motor 13 is provided to drive the paddle rotor at a speed variable from 400 to 1500 revolutions per minute.

The turbocooler R essentially comprises a cylindrical tubular body 1', preferably disposed with its axis horizontal, closed at its opposite ends 2'3' and provided with a coaxial cooling jacket 4' intended to be traversed, for example, by a refrigerant liquid.

The tubular body 1' is provided with an inlet opening 5' for the material treated in the turbosteriliser A and a cooled material discharge opening 6'.

A paddle rotor 9' is rotatably supported in the tubular body 1', the paddles 10' of which are disposed helically and are oriented to centrifuge and simultaneously convey the material subject to treatment towards the outlet.

A motor 13' is provided for driving the rotor 9' at a speed variable from 400 to 100 revolutions per minute.

The high pressure steriliser SP is of conventional type and is in communication with the turbosteriliser A via a suction-blower unit 8 and, on the opposite side, with the condenser C. This latter, which is of conventional type, is in turn in contact with the turbocooler R through a pump P.

Advantageously the delivery duct of the pump P is in communication with the interior of the turbocooler R via a liquid inlet aperture 11 provided close to the inlet opening 5' for the sterilised comminuted material coming from the turbosteriliser A.

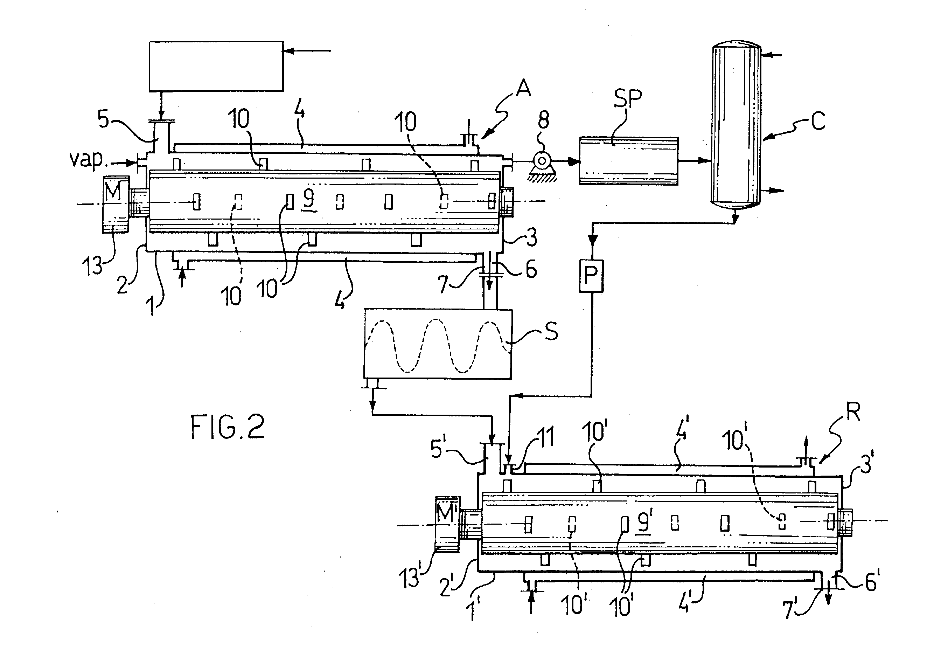

With reference to Figure 2, in which all the same components as those already described have the same reference numerals, the apparatus used in a variant of the method of the invention includes a stabiliser S maintained at a temperature lying between 100 and 300°C, which acts to determine the time for which the product leaving the turbosteriliser A remains at high temperature.

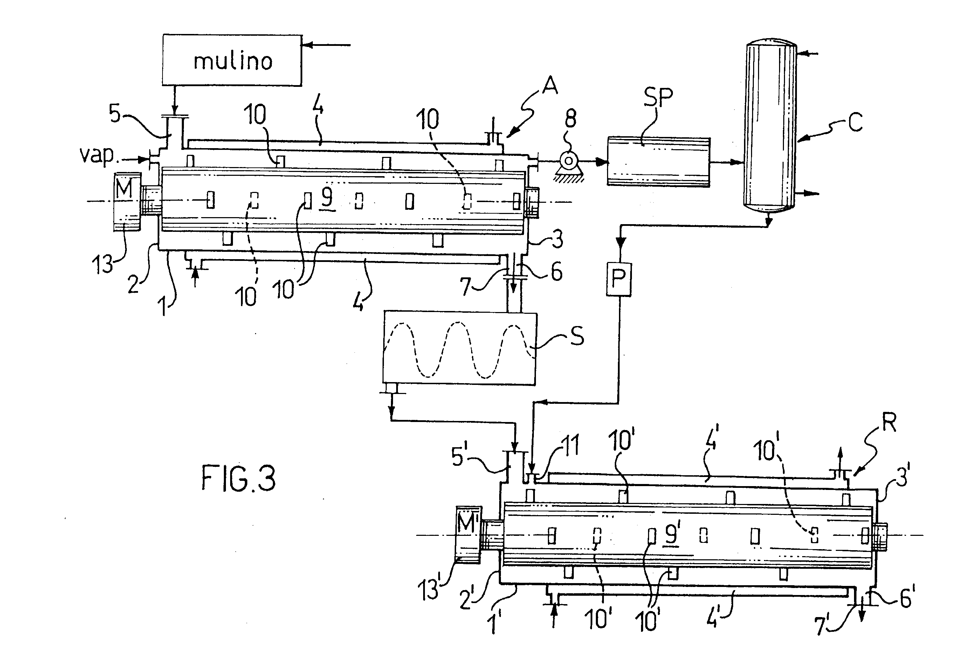

With reference to Figure 3, the installation for disposal of pathogen-containing refuse comprises, in addition to the apparatus already illustrated in the preceding Figures, an indicated with the same reference numerals, a mill of conventional type, for example a multiple mill, the output product of which is continuously fed to the turbosteriliser A.

According to the method of this invention the hospital refuse is subjected wholly (that is to say all of the components of which it is composed) to a forced grinding in an appropriate mill until it reaches an appropriate grain size such that it can be pumped or conveyed in a screw conveyor; in general, and preferably, these components, after comminution, have an almost powder-like consistency, with a moisture content variable from 5 to 30% by weight.

A continuous flow of the various different comminuted components thus obtained is fed continuously to the turbosteriliser A (through the inlet aperture 5 thereof) from the input of which it is taken and mechanically "worked" by the paddles of the rotor 9, which are maintained at an appropriate speed of rotation.

The speed of rotation of this paddle rotor 9 is chosen in such a way that from the inlet of the turbosteriliser A the comminuted material is centrifuged against the hot internal wall of the turbosteriliser itself and accelerated (with respect to the speed of flow at the inlet) towards the outlet so as to be "transformed" from a solid vein flow into a thin tubular layer dynamic in substantial contact with the hot wall of the turbosteriliser, dynamic in that it is continuously displaced towards the outlet opening; the comminuted material is maintained in a highly turbulent state within the said thin tubular layer by the mechanical action of the paddles of the rotor.

It is thus apparent that all of the individual particles of comminuted material are indiscriminately brought into contact with the hot wall of the turbosteriliser for a very large but indefinite number of times, thereby undergoing a corresponding number of heat exchanges, just as corresponding heat exchanges are experienced by the remaining particles of the thin layer. Consequently the sterilisation heat treatment is guaranteed for each particle of this thin layer contrary to what occurs in the bulk treatment of hospital refuse in the prior art.

It has been established that wall temperatures of between 200 and 500°C, and preferably between 350 and 400°C, are more than sufficient for a total sterilisation of hospital refuse thus treated.

It is to be noted that simultaneously with the sterilisation, drying of the (originally wet) comminuted material also takes place with the generation of steam; this steam is more than sufficient to render the material itself non-flammable, thereby eliminating any possible risk of fire.

To this end a flow of steam or inert gas can advantageously be introduced into the turbosteriliser.

The vapours generated within the turbosteriliser, which include steam and substances which evaporate at the sterilisation temperature used, are recovered, for example sucked out from the turbodrier itself and subjected to a sterilisation at high pressure, preferably 10/12 atmospheres, and to condensation.

The sterilised and dry comminuted material at the output of the turbosteriliser can be sent, after cooling, to a conventional discharge.

In accordance with a further characteristic of the present invention this material is supplied to a turbocooler into which the condensed steam mentioned above is simultaneously supplied. In the turbocooler an intimate mixture between the condensate and the sterilised dry comminuted material takes place to obtain a paste which, at the output of the said turbocooler, has a consistency and a temperature allowing it to be easily compacted and briquetted into the form of blocks. These blocks are subsequently discharged or sent to other destinations.

In a preferred embodiment of the invention the material at the output of the turbosteriliser is sent to a stabiliser S in which it is maintained at a temperature lying between 100 and 300°C for a sufficient time, for example 2-10 minutes to guarantee the total elimination of sporogenic germs.

The said stabiliser can be constituted by a static furnace with a timed discharge or, preferably, a low speed screw conveyor system.

The high pressure sterilisation can be replaced by other known sterilisation systems adapted for gases and vapours, such as, for example, those based on ultra violet rays.

In Figure 3 is shown the arrangement of a complete installation for performing the method of the invention which includes a mill, usually of the multiple acting type able to comminute material of diverse consistency, such as, for example, plastics, rubber, cellulose, glass and steel and continuously to supply the turbosteriliser A described above. The installation is completed by a high pressure steriliser SP, a condenser C, a stabiliser S, a turbocooler R and apparatus, not shown, for compaction or briquetting of the product at the output of the turbocooler R.

### EXAMPLE

By utilising the apparatus shown and described above, and following the method of the invention, the material coming from the hospital refuse comminution step in the form of a powder containing an average moisture of about 20% by weight, was continuously supplied into the turbosteriliser A at a flow rate of 300kg per hour, in co-current with a flow of saturated steam.

The wall temperature was controlled about the value of 350°C, whilst the speed of rotation of the paddle rotor was maintained constantly at 850 revolutions per minute. After about three minutes the flow of steam was interrupted, whilst after a delay time of ten minutes dried comminuted material was discharged from the turbosteriliser. This material, having a moisture content of 1.5% by weight and a temperature of 130°C was then fed continuously into the stabiliser S. The said material was maintained at a temperature of 150°C for ten minutes before being discharged and fed continuously into the turbocooler R.

The vapours generated in the turbosteriliser, aspirated out from it were subjected to a pressure of twelve atmospheres in the high pressure steriliser SP and condensed in the condenser C.

The sterilised and stabilised comminuted material was then intimately mixed with the sterile liquid condensate coming from the condenser C in the turbocooler R.

The wall temperature in the turbocooler R was about 0°C, whilst the speed of rotation of the paddle rotor was maintained constantly at 650 revolutions per minute. After a delay of about ten minutes a paste was discharged from the turbocooler R which was subsequently sent to compression in the compactor.

A micro biological examination conducted on samples taken both from the output product from the turbocooler and the product output from the turbosteriliser, after incubation at 20° and at 37° respectively, both in aerobic and in anaerobic conditions, detected absolutely no development of microbic forms.

The invention thus conceived can be subject to variations and modifications, all lying within the ambit of protection thereof. It remaining understood that the critical fundamental condition of the method of this invention for the continuous sterilisation of hospital refuse is constituted by the heat treatment of this refuse reduced to comminuted material of appropriate grain size in a thin and dynamic layer, many variations can be introduced thereto even at the level of the composition of the said comminuted material, such as the chemical/physical quantities in play, and the structural characteristics of the apparatus, all as a function of the particular and contingent requirements which it is intended to attribute to the final product, beyond those specifically aimed at by the present invention. Thus, for example, additives such as binding substances can be added for the purpose of modifying the characteristics of the final product.

## Claims

1. A method for the disposal of hospital refuse and similar refuse which contains pathogenic components, characterised in that it comprises the steps of:
- comminution of the refuse to obtain a finely divided material which can be pumped;
- sterilisation heat treatment of the said comminuted material disposed in a thin layer in contact with a heated wall and maintained in a highly turbulent condition;
- cooling of the sterilised comminuted material and disposal thereof by discharge.

2. A method for disposal of hospital refuse and like refuse including pathogenic components, characterised in that it comprises the steps of:
- comminution of the said refuse to obtain a comminuted material which can be pumped;
- feeding a continuous flow of she said comminuted material to the input of a turbosteriliser (A) comprising a cylindrical tubular body (1)provided with a heating jacket(4) and inlet and outlet apertures (5,6) for material to be treated and treated material respectively, and a paddle rotor (9) rotatably supported within the cylindrical body (1) and driven to rotate at 400-1500 revolutions per minute, the said tubular body (1) having internal cylindrical walls heated to a temperature of 200 to 500°C;
- centrifuging the said comminuted material to form a thin turbulent layer which is caused to flow continuously in contact with the said heated wall towards the discharge opening (6), with simultaneous sterilisation of the said comminuted material;
- cooling and recovering the said comminuted material and subsequently delivering it to a discharge.

3. A process according to Claim 2, characterised in that a flow of saturated steam is introduced into the said turbosteriliser (A) in co-current with the continuous flow of comminuted material to be sterilised.

4. A process according to Claim 2, characterised in that the said sterilised comminuted material at the output of the said turbosteriliser (A) is subjected to heat sterilisation at 100 to 300°C before being sent to the said cooling and recovery stage.

5. A process according to any of claims from 2 to 4, characterised in that simultaneously with sterilisation of the comminuted material it is dried with a corresponding generation of steam.

6. A process according to any of claims 2 to 5 characterised in that the steam present at the interior of the turbosteriliser (A) is aspirated from it, subjected to sterilisation at a pressure of 10-12 atmospheres and condensed.

7. A process according to Claim 6, characterised in that the said sterilised comminuted material at the output of the said turbosteriliser (A) is mixed with the said condensed steam to obtain a paste which is subsequently subjected to briquetting and than delivered to a discharge.

8. A process according to Claim 7, characterised in that the said mixing is performed in a turbocooler (R) comprising a cylindrical tubular body (1') provided with a cooling jacket (4') and an inlet opening (5') for the sterilised comminuted material from the said turbosteriliser (A), and condensed steam, and a discharge opening (6') for the paste formed therein, and a paddle rotor (9') rotatably supported within the cylindrical body (1') and driven to rotate at between about 100 to 200 revolutions per minute.

## Patentansprüche

1. Verfahren zur Vernichtung von Krankenhausabfällen oder ähnlichen Abfällen, welche pathogene Bestandteile enthalten, dadurch gekennzeichnet, daß das Verfahren aus folgenden Schritten besteht:
- Zerkleinerung der Abfälle, um ein fein zerteiltes, pumpfähiges Material zu erhalten
- Sterilisationswärmebehandlung des zerkleinerten Materials, welches in einer dünnen Schicht verteilt in Kontakt mit einer erhitzten Wand gebracht und unter hochturbulenten Bedingungen gehalten wird;
- Abkühlen des sterilisierten zerkleinerten Materials und Entsorgung durch Verwerfung.

2. Verfahren zur Vernichtung von Krankenhausabfällen oder ähnlichen Abfällen, welche pathogene Bestandteile enthalten, dadurch gekennzeichnet, daß das Verfahren aus folgenden Schritten besteht:
- Zerkleinerung der Abfälle, um ein zerteiltes, pumpfähiges Material zu erhalten;
- Speisung eines kontinuierlichen Flusses des zerkleinerten Materials zu dem Einlaß eines Turbosterilisators (A) bestehend aus einem zylindrischen, rohrförmigen Körper (1), welcher mit einem Heizmantel (4) und einer Einlaßöffnung und einer Auslaßöffnung (5,6) für zu behandelndes Material bzw. behandeltes Material versehen ist, und einem Schaufelrotor (9), der innerhalb des zylindrischen Körpers (1) drehbar angebracht und angetrieben wird, um bei 400-1500 Umdrehungen pro Minute zu rotieren, wobei der rohrförmige Körper interne, zylindrische und auf 200 bis 500°C erhitzte Wände besitzt;
- Zentrifugation des zerkleinerten Materials, um eine dünne wirbelnde Schicht zu bilden, welche man kontinuierlich in Kontakt mit den erhitzten Wänden in Richtung der Auslaßöffnung (6) fließen läßt, wobei gleichzeitig die Sterilisation des zerkleinerten Materials erfolgt;
- Abkühlung und Rückgewinnung des zerkleinerten Materials und anschließender Abtransport, um es zu entsorgen.

3. Verfahren nach Anspruch 2, dadurch gekennzeichnet, daß ein gesättigter Wasserdampfstrom zusammenlaufend mit dem kontinuierlichen Fluß des zerkleinerten, zu sterilisierenden Materilas dem Turbosterilisator (A) zugeführt wird.

4. Verfahren nach Anspruch 2, dadurch gekennzeichnet, daß das sterilisierte zerkleinerte Material an dem Auslaß des Turbosterilisators (A) einer Hitzesterilisation bei 100 bis 300°C ausgesetzt wird, bevor es zur Abkühl- und Wiedergewinnungsstufe geschickt wird.

5. Verfahren nach einem der Ansprüche 2 bis 4, dadurch gekennzeichnet, daß gleichzeitig mit der Sterilisation des zerkleinerten Materials dieses mit einer entsprechenden Erzeugung von Dampf getrocknet wird.

6. Verfahren nach einem der Ansprüche 2 bis 5, dadurch gekennzeichnet, daß der im Inneren des Turbosterilisators befindliche Dampf von da angesaugt wird, der Sterilisation bei einem Druck von 10-12 Atmosphären ausgesetzt und kondensiert wird.

7. Verfahren nach Anspruch 6, dadurch gekennzeichnet, daß das sterilisierte zerkleinerte Material an dem Auslaß des Turbosterilisators (A) mit dem kondensierten Dampf gemischt wird, um eine Masse zu erhalten, welche anschließend einem brikettieren ausgesetzt und dann der Entsorgung zugeführt wird.

8. Verfahren nach Anspruch 7, dadurch gekennzeichnet, daß das Mischen in einer Turbokühleinrichtung (R) ausgeführt wird, bestehend aus einem zylindrischen rohrförmigen Körper (11), welcher mit einem Kühlmantel (4') und einer Einlaßöffnung (51) für das sterilisierte zerkleinerte Material aus dem Turbosterilisator (A) versehen ist und kondensiertem Dampf und einer Auslaßöffnung (6') für die darin gebildete Masse und einem innerhalb des zylindrischen Körpers (1') drehbar angebrachten Schaufelrotor (9') und angetrieben, um zwischen ca. 100-200 Umdrehungen pro Minute zu rotieren.

## Revendications

1. Procédé pour éliminer des déchets hospitaliers et déchets analogues qui contiennent des constituants pathogènes, caractérisé en ce qu'il comprend les phases de:
- broyage des déchets pour obtenir une matière finement divisée qui peut être pompée;
- traitement thermique de stérilisation de ladite matière broyée disposée en une mince couche en contact avec une paroi chauffée et maintenue dans un état fortement turbulent;
- refroidissement de la matière broyée stérilisée et élimination de cette matière par déchargement.

2. Procédé pour éliminer des déchets hospitaliers et déchets analogues qui contiennent des constituants pathogènes, caractérisé en ce qu'il comprend les étapes de :
- broyage desdits déchets pour obtenir une matière broyée qui peut être pomp :
- acheminement d'un flux continu de la dite matière broyée à l'entrée d'un turbostérilisateur (A) comprenant un corps tubulaire cylindrique (1) muni d'une chemise chauffante (4) et d'ouvertures d'entrée et de sortie (5, 6) pour la matière à traiter et la matière traitée respectivement, et un rotor à palettes (9) supporté rotatif dans le corps cylindrique (1) et entraîné pour tourner à 400-1500 tours par minute, ledit corps tubulaire (1) ayant des parois cylindriques intérieure chauffées à une température de 200 à 500°C;
- centrifugation de ladite matière broyée pour former une couche turbulente mince que l'on fait s'écouler continuellement en contact avec ladite paroi chauffée vers l' ouverture de déchargement (6), avec stérilisation simultanée de ladite matière broyée ;
- refroidissement et récupération de ladite matière broyée et envoi ultérieur de cette matière à une décharge.

3. Procédé selon la revendication 2, caractérisé en ce qu'un flux de vapeur saturée est introduit dans ledit turbo-stérilisateur (A) à co-courant avec le flux continu de matière broyée à stériliser.

4. Procédé selon la revendication 2, caractérisé en ce que ladite matière broyée stérilisée apparaissant à la sortie dudit turbo-stérilisateur (A) est soumise à une stérilisation par la chaleur à 100 à 300°C avant d'être envoyée à ladite étape de refroidissement et de récupération.

5. Procédé selon une quelconque des revendications 2 à 4, caractérisé en ce que, simultanément à la stérilisation de la matière broyée, celle-ci est séchée avec une production correspondante de vapeur.

6. Procédé selon une quelconque des revendications 2 à 5, caractérisé en ce que la vapeur présente à l'intérieur du turbo-stérilisateur (A) est aspirée de celui-ci, soumise à une stérilisation à une pression de 10-12 atmosphères et condensée.

7. Procédé selon la revendication 6, caractérisé en ce que ladite matière broyée stérilisée présente à la sortie dudit turbo-stérilisateur (A) est mélangée à ladite vapeur condensée pour obtenir une pâte qui est ensuite soumise à une transformation en briquettes puis envoyée à une décharge.

8. Procédé selon la revendication 7, caractérisé en ce que ledit mélange est exécuté dans un turbo-refroidisseur (R) comprenant un corps tubulaire cylindrique (1') muni d'une chemise de refroidissement (4'), d'une ouverture d'entrée (5') pour la matière broyée stérilisée arrivant dudit turbo-stérilisateur (A) et pour la vapeur condenséc, et d'une ouverture de déchargement (6') pour la pâte qui s'y est formée, et un rotor à palettes (9') supporté rotatif à l'intérieur du corps cylindrique (1') et entraîné pour tourner entre environ 100 et 200 tours par minute.
